# EUROPEAN PATENT APPLICATION

(11) **EP 1 609 793 A1**
(43) Date of publication of application: **28.12.2005**
(21) Application number: 04721656.9
(22) Date of filing: 18.03.2004
(51) Int. Cl.: C07D 501/22

(54) **NOVEL CRYSTAL OF 7- 2-(2-AMINOTHIAZOLE-4-YL)-2-HYDROXYIMINOA CETAMIDO-3-VINYL-3-CEPHEM-4-CARBOXYLIC ACID (SYN ISOMER) AND METHOD FOR PREPARATION THEREOF**

(30) Priority: 24.03.2003 JP 2003081273
(71) Applicant: ACS DOBFAR S.p.A., 20067 Tribiano (Milano) (IT)
(72) Inventor: IMAI, Eiji, 3750024 (JP); NIWA, Hiroyuki, 3750024 (JP)
(74) Representative: Frignoli, Luigi
(86) International application number: PCT/JP2004/003622
(87) International publication number: WO 2004/085443

(57) **Abstract**

A novel crystal (B-type crystal) of 7-[2-(2-aminothiazol-4-yl)-2-hydroxyiminoacetamide-3-vinyl-3-cephem-4-carboxylic acid (a syn isomer), **characterized in that** it exhibits peaks at diffraction angles shown in the following Table 1, in its powder X ray diffraction pattern:

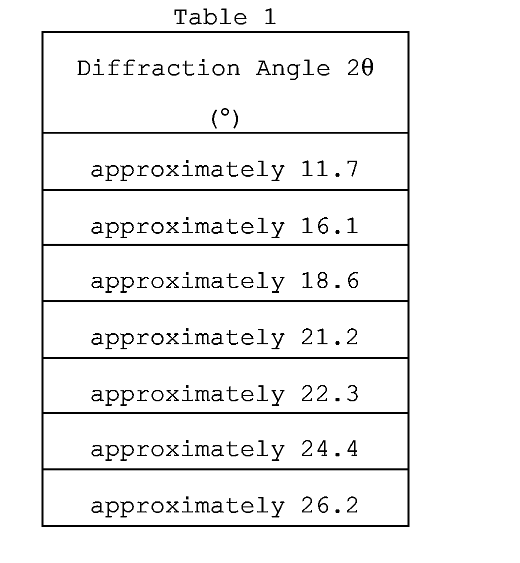

This crystal is obtained by forming a crystal from a solution at a temperature of -5 to 5°C in an acidic state. The crystal is not bulky, exhibits good stability and good filterability, and is excellent in the solubility toward water, and thus can be prepared with case.

## Description

### Technical Field

The present invention relates to a novel crystal of 7-substituted-3-vinyl-3-cephem-4-carboxylic acid which has a wide antibacterial spectrum and is useful as a medicine such as an antibacterial agent and the like, and a method for preparation thereof. More specifically, the present invention relates to a novel crystal of 7-[2-(2-aminothiazol-4-yl)-2-hydroxyiminoacetamide]-3-vinyl -3-cephem-4-carboxylic acid (syn isomer) which is useful as a medicine such as an antibacterial agent and the like, and a method for preparation thereof.

### Background Art

7-[2-(2-aminothiazol-4-yl)-2-hydroxyiminoacetamide]-3-vinyl-3-cephem-4-carboxylic acid (syn isomer) according to the present invention is a compound of the following chemical formula (I) (hereinafter referred to as "compound (I)" in this specification) .

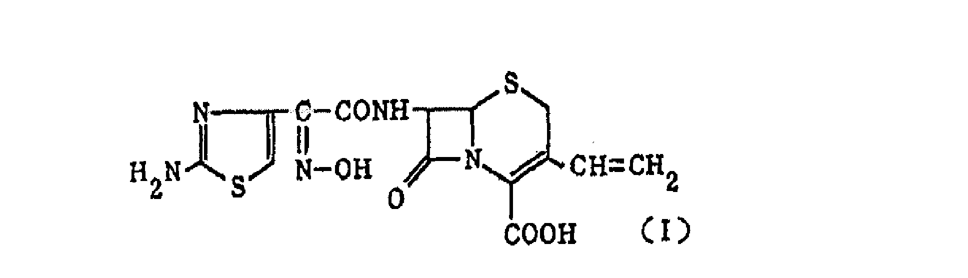

This compound (I) is a known compound useful as a medicine such as an antibacterial agent and the like and used as "CEPHDINYL", name of Japanese Pharmacopoeia. Specifically regarding this compound (I), for example, Japanese Patent Application Publication (JP-B) No. 1-49273 already published describes a chemical structure, various properties and the like thereof, and a method for preparation thereof and the like are describes as examples, further, JP-B No. 6-74276 confirms that the compounds described here are amorphous compounds from subsequent studies of the same applicant. However, these amorphous compounds have problems of high bulk, low purity, instability, small filtration speed and the like, consequently, there are problems of inadequacy as a medicine, poor handling from the standpoint of drug preparation and preservation, and poor handling also in large scale synthesis. Developers of the above-mentioned compound (I) describe a compound of A type crystal as a substance of a novel crystal form of the compound (I) in JP-B NO. 6-74276, as that solving the above-mentioned problems.

The amorphous compound (I) has high bulk, low purity, instability, and small filtration speed and poor handling from the standpoint of drug preparation and preservation, and the compound (I) of the above-mentioned A type crystal has improved these problems, however, these properties are still not sufficiently satisfactory. The present invention has an object of providing a compound (I) of novel crystal form which has further improved these problems, shows excellent solubility particularly in water, and requires a simple preparation process.

### Disclosure of the Invention

The present inventors have intensively investigated to solve such problems, and resultantly succeeded in obtaining a novel crystal of compound (I) which has more excellent solubility in water than that of A type crystal already known, requires a simpler preparation process, and having a novel crystal form, completing the present invention (hereinafter, this compound (I) having a novel crystal form is referred to as "B type crystal of compound (I)").

Namely, the present invention essentially has the following contents.
(1) A novel crystal of 7-[2-(2-aminothiazol-4-yl)-2-hydroxyiminoacetamide]-3-vinyl -3-cephem-4-carboxylic acid (syn isomer), characterized in that it exhibits peaks at diffraction angles shown in the following Table 1, in its powder X ray diffraction pattern: 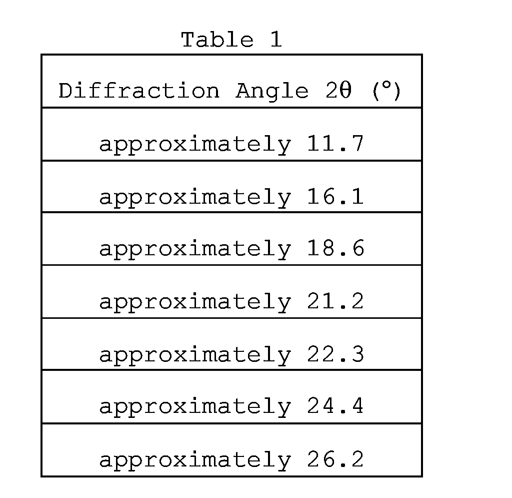
(2) The novel crystal of 7-[2-(2-aminothiazol-4-yl)-2-hydroxyiminoacetamide]-3-vinyl -3-cephem-4-carboxylic acid (syn isomer) according to (1), obtained by crystallization in an acidic state of a solution containing 7-[2-(2-aminothiazol-4-yl)-2-hydroxyiminoacetamide]-3-vinyl -3-cephem-4-carboxylic acid (syn isomer) in a temperature range of -5°C to 5°C .
(3) The novel crystal of 7-[2-(2-aminothiazol-4-yl)-2-hydroxyiminoacetamide]-3-vinyl -3-cephem-4-carboxylic acid (syn isomer) according to (1) or (2), wherein the solution containing 7-[2-(2-aminothiazol-4-yl)-2-hydroxyiminoacetamide]-3-vinyl -3-cephem-4-carboxylic acid (syn isomer) is an aqueous solution of an alkali metal salt of the compound.
(4) The novel crystal of 7-[2-(2-aminothiazol-4-yl)-2-hydroxyiminoacetamide]-3-vinyl -3-cephem-4-carboxylic acid (syn isomer) according to any of (1) to (3), obtained by controlling pH of an aqueous sodium hydrogen carbonate solution of 7-[2-(2-aminothiazol-4-yl)-2-hydroxyiminoacetamide]-3-vinyl -3-cephem-4-carboxylic acid (syn isomer) at from 1 to 3 while cooling the solution in a temperature range from -5°C to 5°C.
(5) A method for preparing a novel crystal of 7-[2-(2-aminothiazol-4-yl)-2-hydroxyiminoacetamide]-3-vinyl -3-cephem-4-carboxylic acid (syn isomer), comprising acidifying a solution containing 7-[2-(2-aminothiazol-4-yl)-2-hydroxyiminoacetamide]-3-vinyl -3-cephem-4-carboxylic acid (syn isomer) in a temperature range from -5°C to 5°C to cause formation of a crystal.
(6) The method for preparing a novel crystal of 7-[2-(2-aminothiazol-4-yl)-2-hydroxyiminoacetamide]-3-vinyl -3-cephem-4-carboxylic acid (syn isomer) according to (5), wherein the acidic state of the solution includes pH values of 1 to 3.
(7) The method for preparing a novel crystal of 7-[2-(2-aminothiazol-4-yl)-2-hydroxyiminoacetamide]-3-vinyl -3-cephem-4-carboxylic acid (syn isomer) according to (5) or (6), wherein the solution containing 7-[2-(2-aminothiazol-4-yl)-2-hydroxyiminoacetamide]-3-vinyl -3-cephem-4-carboxylic acid (syn isomer) is an aqueous solution of an alkali metal salt of the compound.
(8) The method for preparing a novel crystal of 7-[2-(2-aminothiazol-4-yl)-2-hydroxyiminoacetamide]-3-vinyl -3-cephem-4-carboxylic acid (syn isomer) according to any of (5) to (7), wherein the temperature of the solution under an acidic state is 0°C to 2°C.
(9) A method for preparing an anhydrous form of a novel crystal of 7-[2-(2-aminothiazol-4-yl)-2-hydroxyiminoacetamide]-3-vinyl -3-cephem-4-carboxylic acid (syn isomer), wherein a novel crystal obtained by the method according to any of (5) to (8) is further frozen at temperatures from -5°C to -80°C, and then subjected to vacuum drying.
(10) The method for preparing an anhydrous form of a novel crystal of 7-[2-(2-aminothiazol-4-yl)-2-hydroxyiminoacetamide]-3-vinyl -3-cephem-4-carboxylic acid (syn isomer) according to (9), wherein the conditions for vacuum drying include a degree of vacuum of 0.1 to 0.001 mmHg and a temperature of -20 to 35°C.

### Brief Description Of Drawings

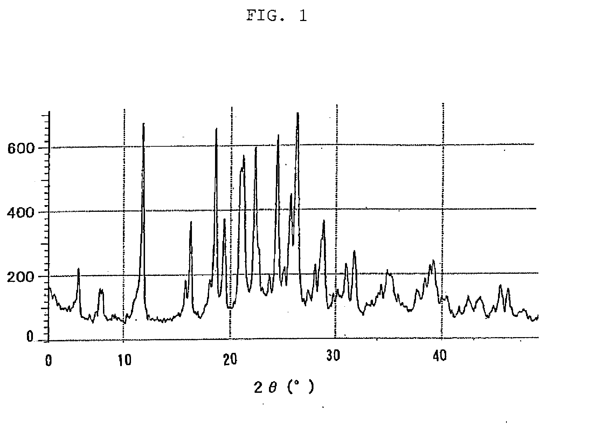

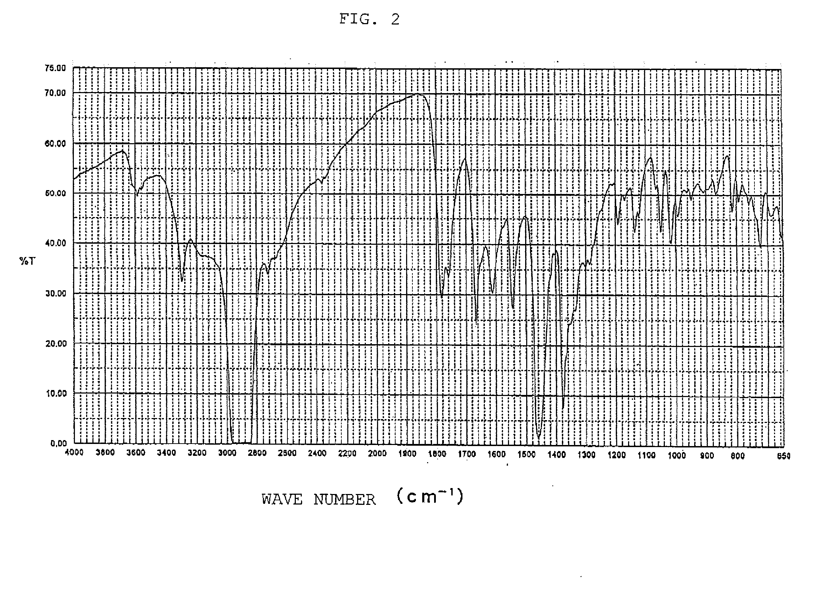

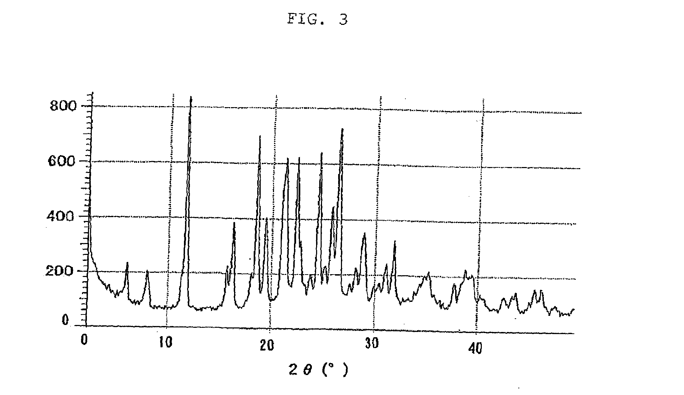

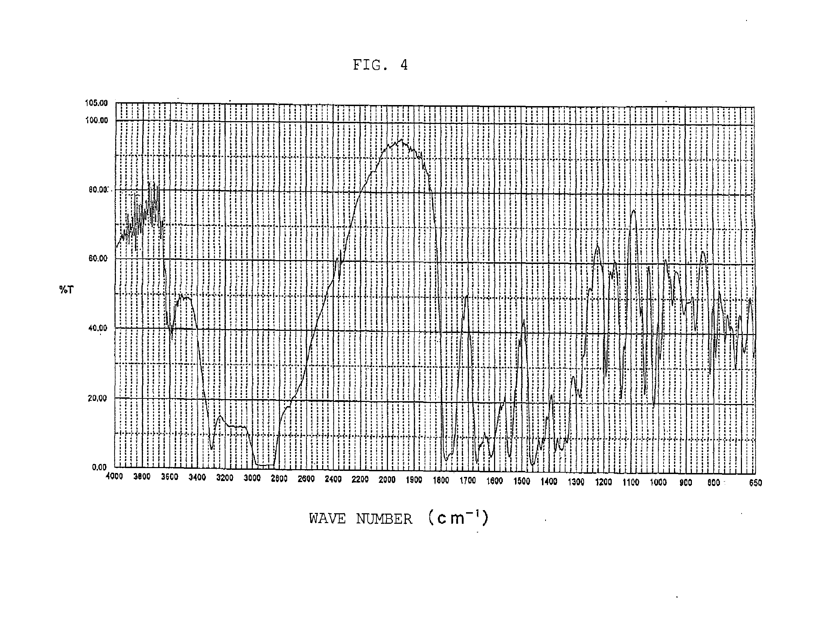

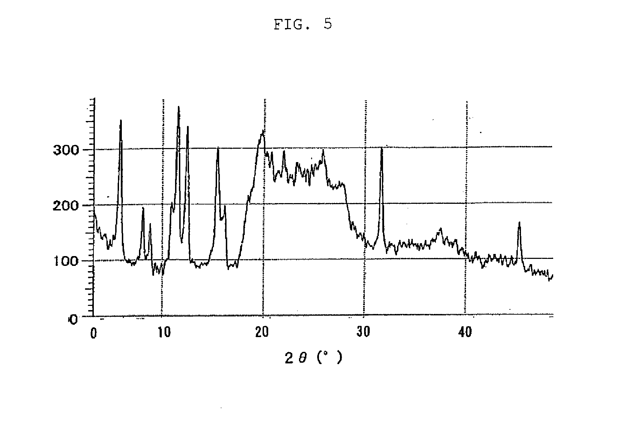

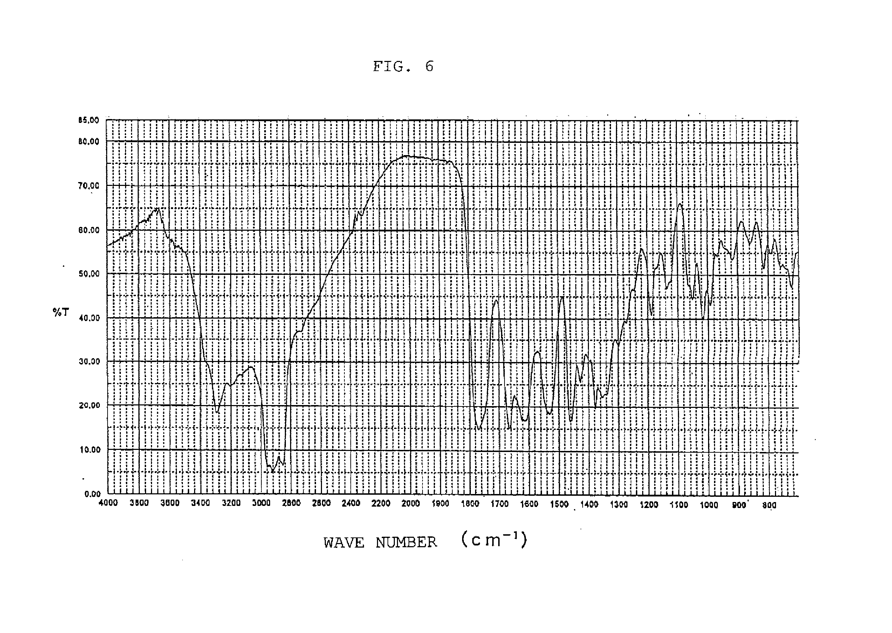

Fig. 1 is an X ray diffraction chart of a mono-hydrate of a B type crystal of a compound (I) of the present invention, and Fig. 2 is a chart of an infrared absorption spectrum of a mono-hydrate of a B type crystal of a compound (I) of the present invention. Fig. 3 is an X ray diffraction chart of an anhydrous form of a B type crystal of a compound (I) of the present invention, and Fig. 4 is a chart of an infrared absorption spectrum of an anhydrous form of a B type crystal of a compound (I) of the present invention. Fig. 5 is an X ray diffraction chart of an anhydrous form of a crystal of a compound (I) obtained in Reference Example, and Fig. 6 is a chart of an infrared absorption spectrum of an anhydrous form of a crystal of a compound (I) obtained in Reference Example.

### Best Modes For Carrying Out The Invention

The physicochemical properties of a B type crystal, novel crystal of 7-[2-(2-aminothiazol-4-yl)-2-hydroxyiminoacetamide]-3-vinyl -3-cephem-4-carboxylic acid (syn isomer) obtained by the present invention, and a method for preparation thereof, are described in detail below.

### 1) Powder X ray diffraction pattern of B type crystal

A B type crystal obtained by the above-mentioned preparation method of the present invention is, before its freeze drying treatment, a crystal of a mono-hydrate of a compound (I), and after its freeze drying treatment, a crystal of a 1/4-hydrate or anhydrous form of a compound (I).

This B type crystal which is a novel crystal of a compound (I) shows characteristic peaks at diffraction angles (2θ (°)) shown in the following Table 2, in its powder X ray diffraction pattern:

**Table 2**

| Diffraction Angle 2θ (°) | Relative intensity |
|---|---|
| approximately 11.7 | 100 |
| approximately 16.1 | 49 |
| approximately 18.6 | 94 |
| approximately 21.2 | 74 |
| approximately 22.3 | 79 |
| approximately 24.4 | 82 |
| approximately 26.2 | 95 |

Such a B type crystal which is a novel crystal of a compound (I) specified by the above-mentioned X ray diffraction pattern in Table 2 has an X ray diffraction pattern and positions of peaks apparently different from those of the above-mentioned A type crystal of 7-[2-(2-aminothiazol-4-yl)-2-hydroxyiminoacetamide]-3-vinyl -3-cephem-4-carboxylic acid (syn isomer) described in JP-B No. 6-74276, and thus is a compound having a novel crystal structure.

As one specific example showing such a powder X ray diffraction pattern, a powder X ray diffraction pattern of a mono-hydrate of a B type crystal of a compound (I) obtained in Example 1 described later is shown in Fig. 1 and a powder X ray diffraction pattern of an anhydrous form of a B type crystal of a compound (I) obtained in Example 2 is shown in Fig. 3. However, these diffraction patterns are only mentioned as a reference, and any crystals of a mono-hydrate or an anhydrous form of a compound (I) having its powder X ray diffraction pattern showing characteristic peaks substantially at the same positions as the above-mentioned diffraction pattern are identified as the B type crystal of a compound (I) of the present invention which is a novel crystal.

### 2) Infrared absorption spectrum

As one example of data of an infrared absorption spectrum, an infrared absorption spectrum of a mono-hydrate of a B type crystal of a compound (I) obtained in Example 1 described later is shown in Fig. 2 and an infrared absorption spectrum of an anhydrous form of a B type crystal of a compound (I) obtained in Example 2 is shown in Fig. 4. However, these spectra are only mentioned as a reference, and any crystals of a mono-hydrate or an anhydrous form of a compound (I) having infrared absorption spectra which are substantially the same as these spectra are identified as the B type crystal of a compound (I) which is a novel crystal.

### 3) Method of preparing B type crystal of compound (I)

7-[2-(2-aminothiazol-4-yl)-2-hydroxyiminoacetamide]-3-vinyl-3-cephem-4-carboxylic acid (syn isomer) prepared by conventionally known methods, for example, a method described in JP-B 1-49273, is amorphous, and a compound prepared by a method described in JP-B No. 6-74276 is a compound of A type crystal, however, according to the method of the present invention, a compound of B type crystal which is a novel crystal different from them is obtained.

According to the method of the present invention, a mono-hydrate of a B type crystal of a compound (I) can be obtained by crystallizing a solution containing 7-[2-(2-aminothiazol-4-yl)-2-hydroxyiminoacetamide]-3-vinyl -3-cephem-4-carboxylic acid (compound I) at a temperature in a range from -5°C to 5°C in an acidic state.

Preferable examples of the solution containing the compound (I) used here include those obtained by dissolving the compound (I) in an aqueous solution of an alkali metal salt such as sodium carbonate, sodium hydrogen carbonate, potassium carbonate and the like. The concentration of an alkali metal salt on this aqueous solution is preferably 1 to 20 wt%, further preferably 1 to 5 wt%. This solution of the compound (I) is, further if necessary, subjected to column chromatography with activated carbon, nonionic adsorption resin, alumina, acidic aluminum oxide and the like, then, an acidic substance such as hydrochloric acid, phosphoric acid and the like is added to control pH of the solution in an acidic range from 1 to 3, causing formation of a crystal of a mono-hydrate of the compound (I). It is necessary that this acidifying operation is conducted at a temperature in a range from -5°C to 5°C. Crystal formation conditions by this acidification include, further preferably, a pH of 1.5 to 2.5 and a temperature of 0°C to 2°C. While maintaining such conditions, a solution containing the compound (I) is stirred gently for 0.5 to 2 hours, to form a crystal. This crystal is filtrated by an ordinary method, and dried at 25°C to 30°C under reduced pressure. By the method described above, a mono-hydrate of a B type crystal of the compound as described above is obtained. The water content of the resultant mono-hydrate of a B type crystal of the compound (I) is approximately 4 to 4.5 wt% measured by the Karl Fischer method.

Thus obtained mono-hydrate of a B type crystal of the compound (I) can be frozen at temperatures from -5 to -80°C, preferably -10 to -30°C, then, vacuum-dried under a reduced pressure of 0.1 to 0.001 mmHg, preferably 0.01 to 0.001 mmHg for 100 to 200 hours, to obtain a 1/4-hydrate or anhydrous form of a B type crystal of a compound (I) from which crystallization water has been stripped. The temperature in vacuum drying is -20 to 35°C, preferably -15 to 30°C, most preferably -15 to 25°C. By selecting freeze vacuum drying conditions, various hydrates between a 1/4-hydrate and an anhydrous form of a B type crystal of a compound (I) can be obtained.

Next, the method for preparing a compound (I), raw material used in the present invention will be illustrated.

Though a compound (I) or its salts can be prepared by the method described in JP-B No. 1-49273 as described above, a method according to the following reaction scheme is preferable for obtaining a compound (I) particularly in high yield.

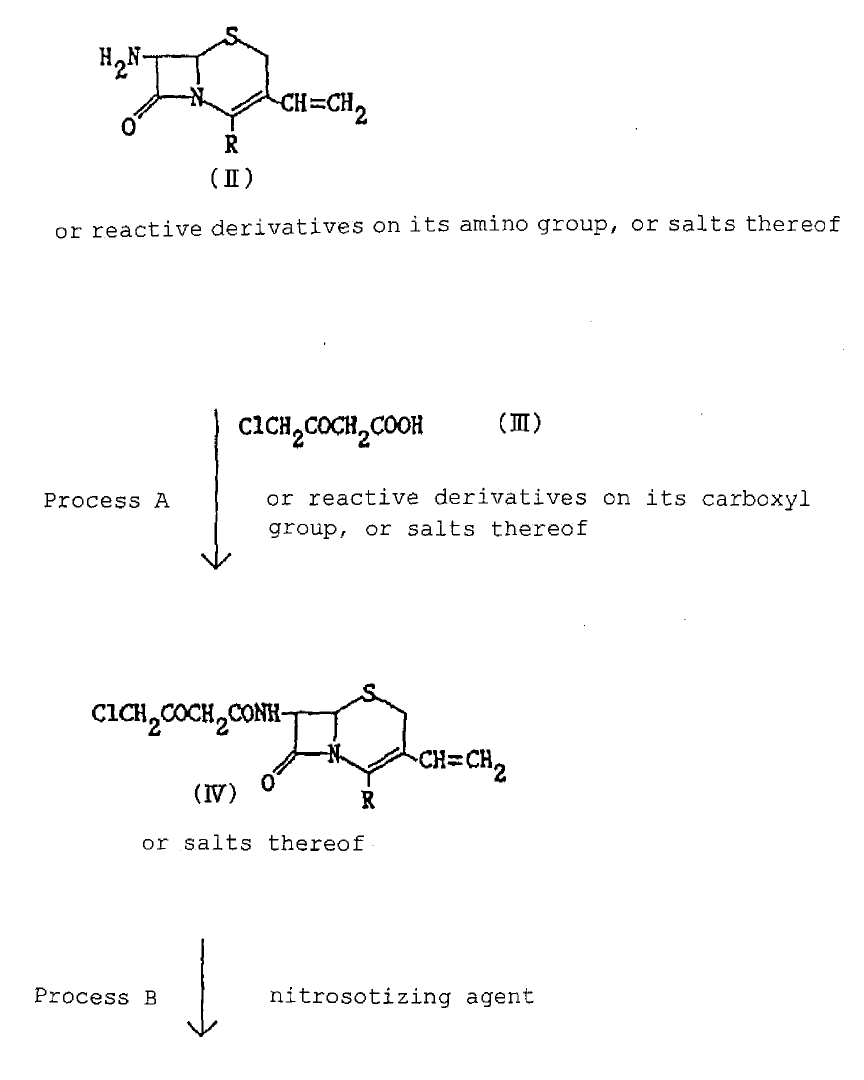

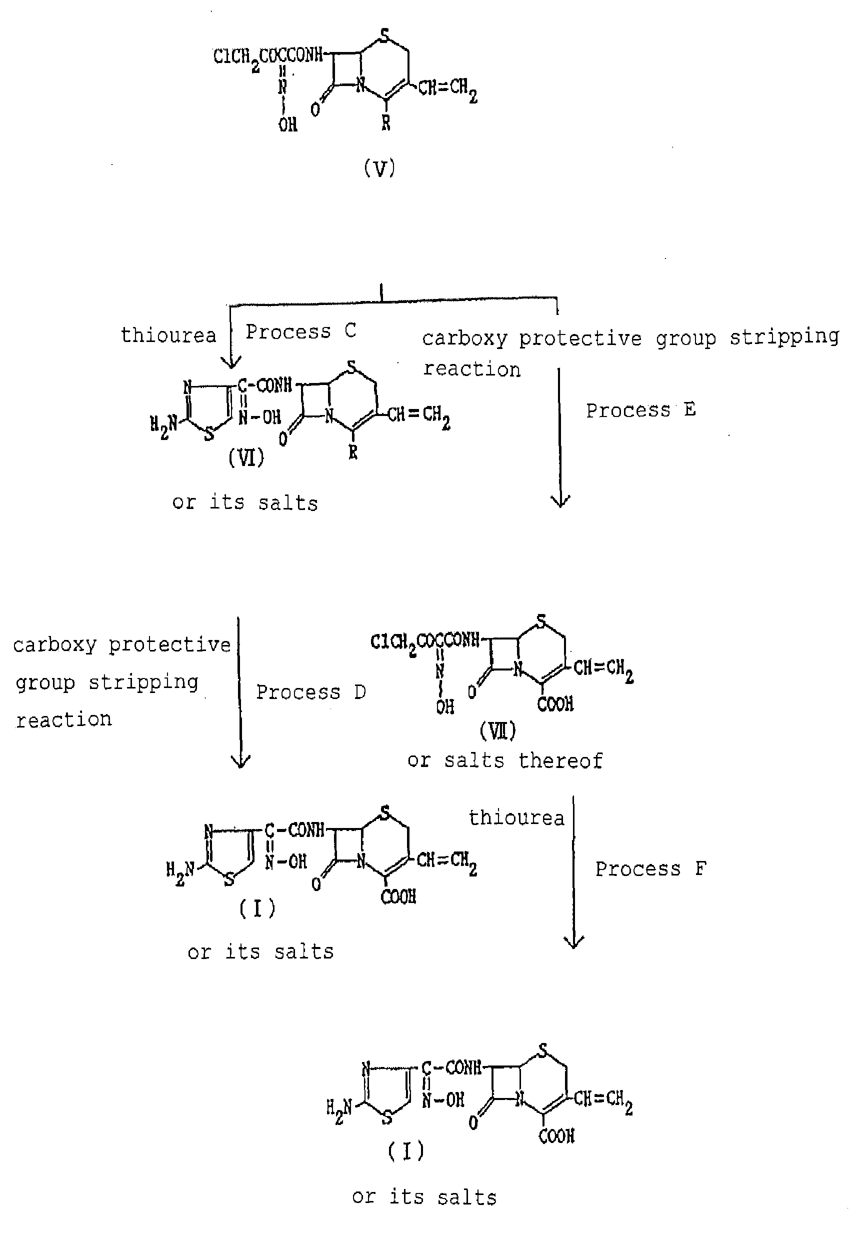

In this reaction formula, R means "protected carboxyl group", and as suitable protected carboxyl groups R, those usually used in the field of cephalosporin compounds are mentioned, and examples thereof include esterified carboxyl groups. Preferable examples of the "esterified carboxyl groups" include ar (lower) alkoxycarbonyl groups and the like such as a benzyloxycarbonyl group, benzhydriloxycarbonyl group, trityloxycarbonyl group and the like.

Suitable salts of a compound (I) are conventional avirulent salts such as salts with bases, acid-addition salts and the like, and examples thereof include alkali metal salts such as sodium salts, potassium salts and the like; alkaline earth metal salts such as calcium salts, magnesium salts and the like; salts with inorganic bases such as ammonium salts and the like; salts with organic bases such as organic amines such as triethylamine salts, pyridine salts, picoline salts, ethanolamine salts, triethanolamine salts, dicyclohexylamine salts, N,N'-dibenzylethylenediamine salts and the like; inorganic acid-addition salts such as hydrochloric acid salts, hydrobromic acid salts, sulfuric acid salts, phosphoric acid salts and the like; organic carboxylic acid-addition salts or organic sulfonic acid-addition salts such as formic acid salts, acetic acid salts, trifluoroacetic acid salts, maleic acid salts, tartaric acid salts, methanesulfonic acid salts, benzenesulfonic acid salts, p-toluenesulfonic acid salts and the like; organic acid-addition salts such as 3-(N-formyl-N-hydroxyamino)propylphosphonic acid salts, 2-hydroxy-3-(N-hydroxyamino)propylphosphonic acid salts and the like; salts with basic amino acids or acidic amino acids such as arginine, aspartic acid, glutamic acid and the like.

Next, the method for preparing a compound (I) will be illustrated in detail with the above-mentioned reaction scheme.

First, a compound (IV) of the process A or salts thereof can be prepared by reacting a compound (II) or a reactive derivative on its amino group, or salts thereof with a compound (III) or a reactive derivative on its carboxy group, or salts thereof.

As the reactive derivative on its amino group of a compound (II), mentioned are conventional derivatives, for example, silyl derivatives produced by reaction of a compound (II) with a silyl compound such as trimethylsilylacetamide, bis(trimethylsilyl)acetamide, bis (trimethylsilyl) urea and the like, and as the reactive derivative of a compound (III), mentioned are acid halides such as acid chlorides, acid bromies and the like, and these acid halides can be produced by reaction of diketenes and halogens.

As the salts of a compound (II), examples of the acid-addition salts for a compound (I) are mentioned as themselves, and as the salts of a compound (III), examples of the salts with bases for a compound (I) are mentioned as themselves.

The reaction is conducted usually in a conventional solvent not exerting a reverse influence on this reaction such as water, acetone, dioxane, acetonitrile, chloroform, benzene, carbon tetrachloride, methylene chloride, ethylene chloride, tetrahydrofuran, ethyl acetate, N,N-dimethylformamide, N,N-dimethylacetamide, pyridine, hexamethylphosphoramide and the like, or a mixture thereof. The reaction temperature is not particularly limited, and the reaction is conducted usually under cooling or heating.

A compound (V) in the process B can be produced by reaction of a compound (IV) or salts thereof with a nitrosotizing agent.

The nitrosotizing agent include nitrous acid and derivatives thereof, nitrosyl halides such as nitrosyl chloride, nitrosyl bromide and the like, alkali metal nitrites such as sodium nitrite, potassium nitrite and the like, alkyl nitrites such as butyl nitrite, pentyl nitrite, isoamyl nitrite and the like. When a salt of nitrous acid, for example, its alkali metal salt is used as the nitrosotizing agent, it is preferable to effect the reaction in the presence of an inorganic or organic acid such as, for example, hydrochloric acid, sulfuric acid, forming acid, acetic acid and the like.

This reaction is conducted usually in a conventional solvent not exerting a reverse influence on this reaction such as water, acetic acid, benzene, methanol, ethanol, tetrahydrofuran, methylene chloride and the like, or a mixture thereof. The reaction temperature is not particularly limited, and the reaction is conducted usually under cooling or heating. A compound (V) can also be used as a raw material of the subsequent process C without isolation and purification.

A compound (VI) in the process C or salts thereof can be prepared by reacting a compound (V) with thiourea. This reaction is conducted usually in a conventional solvent not exerting a reverse influence on this reaction such as ethyl acetate, methylene chloride, chloroform, carbon tetrachloride, tetrahydrofuran, N,N-dimethylformamide, N,N-dimethylacetamide, dioxane, water, acetic acid, formic acid and the like, or a mixture thereof. The reaction temperature is not particularly limited, and the reaction is conducted usually under cooling or heating.

A compound (I) in the process D or salts thereof can be prepared by subjecting a compound (VI) or salts thereof to a stripping reaction of a carboxyl protective group. As the salts of a compound (VI), acid-addition salts exemplified for a compound (I) are mentioned as they are.

As the method for stripping a carboxy protective group in this reaction, conventional methods such as hydrolysis, reduction and the like are listed.

### (i) Hydrolysis

Hydrolysis is preferably conducted in the presence of an acid. Examples of such an acid include inorganic acids such as hydrochloric acid, hydrobromic acid, sulfuric acid and the like, organic acids such as formic acid, acetic acid, trifluoroacetic acid, propionic acid, methanesulfonic acid, benzenesulfonic acid, p-toluenesulfonic acid and the like, acidic ion exchange resins and the like. When organic acids such as trifluoroacetic acid and p-toluenesulfonic acid are used among these acids, it is desirable to effect a reaction in the presence of a cation collector such as, for example, anisole and the like. Further, instead of the above-mentioned acids, Lewis acids such as boron trifluoride, boron trifluoride etherate, aluminum trichloride, antimony pentachloride, ferric chloride, stannic chloride, titanium tetrachloride, zinc chloride and the like can also be used, and further, also when a Lewis acid is used, the reaction can be conducted in the presence of a cation collector such as, for example, anisole and the like.

Hydrolysis is conducted usually in a conventional solvent not exerting a reverse influence on this reaction such as methylene chloride, water, methanol, ethanol, propanol, tertiary butyl alcohol, tetrahydrofuran, N,N-dimethylformamide, N,N-dimethylacetamide, dioxane and the like, or a mixture thereof, further, also when the above-mentioned acid is liquid, it can be used as the solvent. The reaction temperature of this hydrolysis is not particularly limited, and the reaction is conducted usually under cooling or heating.

### (ii) Reduction

Reduction is conducted by a conventional method including chemical reduction and catalytic reduction.

Examples of the reducing agent used in chemical reduction include combinations of a metal such as tin, zinc, iron and the like or a metal compound such as chromium chloride, chromium acetate and the like with an organic or inorganic acid such as formic acid, acetic acid, propionic acid, trifluoroacetic acid, p-toluenesulfonic acid, hydrochloric acid, hydrobromic acid and the like.

As the catalyst used in catalytic reduction, there are mentioned conventional catalysts such as platinum catalysts such as platinum plate, platinum sponge, platinum black, colloid platinum, platinum oxide, platinum wire and the like, palladium catalysts such as palladium sponge, palladium black, palladium oxide, palladium carbon, colloid palladium, palladium barium sulfate, palladium barium carbonate and the like, nickel catalysts such as reduced nickel, nickel oxide, Raney nickel and the like, cobalt catalysts such as reduced cobalt, Raney cobalt and the like, iron catalysts such as reduced iron, Raney iron and the like, copper catalysts such as reduced copper, Raney copper, Ullmann copper and the like.

Reduction is conducted usually in a conventional solvent not exerting a reverse influence on this reaction such as water, methanol, ethanol, propanol, N,N-dimethylformamide and the like, or a mixture thereof. Further, when the above-mentioned acid used in chemical reduction is liquid, it can be used as the solvent. As the solvent used in catalytic reduction, conventional solvents such as diethyl ether, dioxane, tetrahydrofuran and the like or mixtures thereof are mentioned, in addition to the above-mentioned solvents. The reaction temperature is not particularly limited, and the reaction is conducted usually under cooling or heating.

A compound (VII) in the process E or salts thereof can be prepared by subjecting a compound (V) to a stripping reaction of a carboxy protective group. As the salts of a compound (VII), salts with bases exemplified for a compound (I) are mentioned. The stripping reaction of a carboxy protective group in this reaction is conducted in the same manner as described in the process D.

A compound (I) in the process F or salts thereof can be produced by reacting a compound (VII) or salts thereof with thiourea. This reaction is conducted in the same manner as the method described in the process C.

When a compound (I) obtained in the above-mentioned method is in isolated form, it can be converted into its salts, particularly, acid-addition salts by an ordinary method. When the resultant compound (I) is in the form of salts, it can be converted into a free compound by an ordinary method.

A compound (I) prepared in the above-mentioned preparation method can be converted into a mono-hydrate of a B type crystal of a compound (I) in the form of novel crystal form by applying the method for preparing a mono-hydrate of a B type crystal of a compound (I) of the present invention already described in its isolation step. Further, this mono-hydrate of a B type crystal of a compound (I) is frozen at temperatures of -5 to -80°C as described already, and vacuum-dried for 100 to 200 hours under a reduced pressure of 0.1 to 0.001 mmHg, preferably 0.01 to 0.001 mmHg. Thus, an anhydrous form or a 1/4-hydrate of a B type crystal of a compound (I) can be obtained. The temperature in vacuum drying is -20 to 35°C, preferably -15 to 35°C, most preferably -10 to 25°C.

The above-mentioned preparation is a method for obtaining a B type crystal of a compound (I) in high yield, and can be conducted with extreme safety. It is also suitable for large scale preparation of a B type crystal of a compound (I).

The anhydrous form or mono-hydrate of a B type crystal which is a novel crystal of a compound (I) of the present invention thus obtained is relatively compact and not bulky, has very high crystal purity, is extremely stable against heat, light and the like, and simultaneously, has excellent solubility in water. Therefore, while a compound (I) is originally a substance useful as an antibacterial agent, an anhydrous form or mono-hydrate of a B type crystal as its novel crystal is, because of excellent physical properties as described above, easy for preparation, suitable for use as a medical preparation, and also gives easy handing in preparation and preservation.

Further, the anhydrous form or mono-hydrate of a B type crystal of a compound (I) has sufficiently large filtration speed, and also has a feature that working on preparation can be conducted very efficiently. Therefore, the anhydrous form or mono-hydrate of a B type crystal of a compound (I) of the present invention is a substance which is extremely suitable also for large scale synthesis such as preparation in a factory. Further, due to easy filtration, impurities are not mixed in easily in a purification process, consequently, a B type crystal of a compound (I) of high quality can be prepared.

As described above, the anhydrous form or mono-hydrate of a B type crystal of a compound (I) of the present invention has extremely excellent merits and is very excellent as compared with conventionally known amorphous compounds (I), and provides a simpler preparation process than an A type crystal of a compound (I) .

The present invention will be further described in detail by preparation examples and examples below. "%" in each example is "wt%" unless otherwise stated.

### Preparation Example 1

A hydrochloride (26.6 kg) of a benzhydryl ester of 7-amino-3-vinyl-3-cephem-4-carboxylic acid was dissolved in N,N-dimethylacetamide (78L) and the solution was cooled down to -10°C.

Separately, a methylene chloride solution of 4-chloroacetoacetic acid chloride obtained by bubbling through of chlorine (6.5 kg) at -25°C or lower was dropped with stirring into a solution prepared by dissolving diketene (7.6 kg) in methylene chloride (130 L) at temperatures of -10 to 0°C. After completion of dropping, stirring was continued for 30 minutes at the same temperature.

After completion of the reaction, methylene chloride (130 L) was added to the reaction liquid with stirring at 5°C, then, a 6% sodium hydrogen carbonate aqueous solution (260 L) was added with stirring at 5°C, then, the organic layer was removed. Then, the organic layer was washed with water (156 L) at 5°C. The organic layer was concentrated under reduced pressure until 182 L, then, acetone (130 L) was added, and concentrated under reduced pressure until 182 L. Acetone (78 L) was added to the concentrated liquid, then, methanol (130 L) was dropped at 20°C. After stirring for 10 minutes, water (260 L) was added and the solution was cooled down to 5°C with stirring, then, allowed to stand still overnight.

The deposited crystal was filtrated, washed with a 30% methanol aqueous solution (130 L), then, dried to obtain a benzhydryl ester (31.3 kg) of 7-(4-chloroacetacetamide)-3-vinyl-3-cephem-4-carboxylic acid.
Melting point: 171°C
IR (Nujol): 3260, 1775, 1713, 1661, 1224, 698cm⁻¹

### Preparation Example 2

A benzhydryl ester (30.8 kg) of 7-(4-chloroacetacetamide)-3-vinyl-3-cephem-4-carboxylic acid was suspended in methylene chloride (290 L) and the suspension was cooled down to -5°C. After cooling, a 10.6 N hydrogen chloride tetrahydrofuran solution (267 ml) was added, then, isoamyl nitrite (7.1 kg) was added, then, the mixture was stirred at 0°C for 60 minutes.

The resulted methylene chloride solution of a benzhydryl ester of 7-(4-chloro-2-hydroxyiminoacetamide)-3-vinyl-3-cephem-4-car boxylic acid was added over a period of 1 hour to a solution prepared by dissolving thiourea (6.5 kg) in N,N-dimethylacetamide (78 L) while effecting a reaction of them under reduced pressure concentration. Methylene chloride was distilled off, then, stirring was continued at 50°C for 30 minutes. After completion of the reaction, acetone (145 L) and a 5% sodium hydrogen carbonate aqueous solution (73 L) were added at 20°C, and this solution was dropped into water (290 L) over a period of 20 minutes while maintaining the temperature at 20°C. After completion of dropping, pH of the solution was controlled to 6 with a 5% sodium hydrogen carbonate aqueous solution, and the resulted solution was cooled to 5°C with stirring, then, allowed to stand still overnight.

The deposited precipitate was filtrated off, washed with a 40% acetone aqueous solution (145 L), then, dried, to obtain an amorphous benzhydryl ester (syn isomer) (36.9 kg) of 7-[2-(2-aminothiazol-4-yl)-2-hydroxyiminoacetamide]-3-vinyl -3-cephem-4-carboxylic acid.
IR (Nujol) : 3320, 1782, 1720, 1670, 1618, 1528, 1220, 698cm⁻¹

### Example 1

25.0 g of the benzhydryl ester (amorphous) of 7-[2-(2-aminothiazol-4-yl)-2-hydroxyiminoacetamide]-3-vinyl -3-cephem-4-carboxylic acid was dissolved in mixed liquid of methylene chloride (150 ml) and anisole (15 ml). Into the resulted solution was dropped 2,2,2-trifluoroacetic acid (500 ml) at 5°C with stirring, then, stirring was continued for 30 minutes.

The reaction liquid was concentrated under reduced pressure to obtain a residue, and diisopropyl ether (250 ml) was added to the residue to obtain a solid substance (16.5 g). This was ground and dissolved in isopropyl alcohol (80 ml) and treated with activated carbon (1.6 g), then, the solution was allowed to stand at 5°C for 3 hours. The resulted deposit was filtrated off, to obtain a colorless crystal (7.8 g) (this crystal includes one molecule of isopropyl alcohol).

This resulted crystal (6.0 g) was added to water (300 ml), and pH thereof was controlled to 6.0 using a saturated aqueous solution of sodium hydrogen carbonate. Then, this solution was cooled to 0°C, and pH thereof was controlled to 2.0 with 4N hydrochloric acid (7 mL) to cause deposition of a crystal from the solution. The resulted crystal was filtrated and dried under reduced pressure at 25°C over a period of 12 hours to obtain a crystal (5.8 g) of 7-[2-(2-aminothiazol-4-yl)-2-hydroxyiminoacetamide]-3-vinyl -3-cephem-4-carboxylic acid (syn isomer).

As a result of thermal analysis of this crystal, a heat absorption peak was observed at 48.7°C and a weight reduction of 6.56% was confirmed. The water content was measured by a Karl Fischer method to find a water content of 4.11% (calculated value as mono-hydrate: 4.36%). From these results, the crystal obtained above was determined as a mono-hydrate.

This crystal was applied to an X ray diffractometer (manufactured by Shimadzu Corp., XRD-6000) under the following measurement conditions, and its powder X ray diffraction pattern was measured.

| | |
|---|---|
| Anticathode: | Cu |
| Filter: | Ni |
| Tube voltage: | 30 kv |
| Tube electric current: | 10 mA |
| Detector: | scintillation counter |

The results of this measurement are shown in Fig. 1 and Table 3.

**Table 3**

| Comparison | Diffraction Angle 2θ (°) | Relative intensity |
|---|---|---|
| ○ | 11.72 | 100 |
| ○ | 16.10 | 49 |
| ○ | 18.55 | 94 |
| ○ | 21.18 | 74 |
| ○ | 22.28 | 79 |
| | 23.62 | 10 |
| ○ | 24.37 | 82 |
| ○ | 26.22 | 95 |
| | 28.00 | 21 |

In Table 3, a peak appended with "○ mark" in the column of comparison denotes a peculiar peak specifying a B type crystal of a compound (I) . Therefore, it was found that the crystal of 7-[2-(2-aminothiazol-4-yl)-2-hydroxyiminoacetamide]-3-vinyl -3-cephem-4-carboxylic acid (syn isomer) obtained in Example 1 above was a mono-hydrate of its B type crystal.

Further, a chart of an infrared absorption spectrum of a novel crystal of the above-mentioned mono-hydrate obtained here is shown in Fig. 2.

IR (Nujol): 1780, 1460 cm⁻¹

### Example 2

Likewise, the crystal deposited from a solution at pH 2.0 in Example 1 was filtrated, and this was preserved overnight at a temperature of -20°C to be frozen, then, dried under vacuum of a degree of vacuum of 0.01 mmHg at room temperature (20°C) for 158 hours, to obtain 21 g of a crystal of
7-[2-(2-aminothiazol-4-yl)-2-hydroxyiminoacetamide]-3-vinyl -3-cephem-4-carboxylic acid (syn isomer). The water content was measured by a Karl Fischer method to find a water content of 1.05%. From these results, the crystal obtained here was determined as an anhydrate of
7-[2-(2-aminothiazol-4-yl)-2-hydroxyiminoacetamide]-3-vinyl -3-cephem-4-carboxylic acid (syn isomer).

This crystal was applied to an X ray diffractometer (manufactured by Shimadzu Corp., XRD-6000) under the following measurement conditions, and its powder X ray diffraction pattern was measured.

| | |
|---|---|
| Anticathode: | Cu |
| Filter: | (Ni) |
| Tube voltage: | 40 kv |
| Tube electric current: | 30 mA |
| Detector: | scintillation counter |

The results of this measurement are shown in Fig. 3 and Table 4.

**Table 4**

| Comparison | Diffraction Angle 2θ (°) | Relative intensity |
|---|---|---|
| ○ | 11.75 | 100 |
| ○ | 16.22 | 41 |
| ○ | 18.60 | 79 |
| | 19.42 | 40 |
| | 21.00 | 54 |
| ○ | 21.24 | 65 |
| ○ | 22.33 | 63 |
| ○ | 24.45 | 65 |
| | 25.70 | 39 |
| ○ | 26.32 | 76 |

In Table 4, a peak appended with "○ mark" in the column of comparison denotes a peculiar peak specifying a B type crystal of a compound (I) . Therefore, it was found that the crystal of 7-[2-(2-aminothiazol-4-yl)-2-hydroxyiminoacetamide]-3-vinyl -3-cephem-4-carboxylic acid (syn isomer) obtained in Example 2 above was an anhydrous form of its B type crystal.

Further, a chart of an infrared absorption spectrum of an anhydrous form of the above-mentioned B type crystal obtained above is shown in Fig. 4.
IR (Nujol): 1770, 1665, 1460 cm⁻¹

### Example 3

The anhydrous form of a B type crystal of 7-[2-(2-aminothiazol-4-yl)-2-hydroxyiminoacetamide]-3-vinyl -3-cephem-4-carboxylic acid (syn isomer) obtained in Example 2, and the A type crystal of a compound (I) obtained by deposition of a crystal from a sodium bicarbonate aqueous solution having a pH value of 3.6 and a temperature of 22°C in Example 1 were used, and the speed of dissolution thereof in water was measured.

Namely, each 10 mg of the above-mentioned A type crystal or B type crystal was put into 50 mL of ion exchanged water and dissolved at a revolution per minute of 60, and the concentrations of each crystal in the solution with the lapse of time were measured by liquid chromatography. The results are shown in Table 5.

**Table 5**

| lapse time (min) | A type crystal concentration (elution %) | B type crystal concentration (elution%) |
|---|---|---|
| 110 | 32.7 | 72.8 |
| 340 | 39.7 | 78.4 |
| 700 | 63.7 | 91.1 |

From these results, it is understood that an anhydrous form of a B type crystal of a compound (I) of the present invention has more excellent solubility in water as compared with a conventional A type crystal, and when used as a medicine, an effect of improving its absorption is expected.

### Reference Example 1

The mono-hydrate of a crystal of 7-[2-(2-aminothiazol-4-yl)-2-hydroxyiminoacetamide]-3-vinyl -3-cephem-4-carboxylic acid (syn isomer) obtained in Example 1 was vacuum-dried under reduced pressure in the presence of phosphorus pentoxide at 60°C for 1 hour, to obtain a crystal of an anhydrous form of
7-[2-(2-aminothiazol-4-yl)-2-hydroxyiminoacetamide]-3-vinyl -3-cephem-4-carboxylic acid (syn isomer).

This crystal was applied to the X ray diffractometer under the same conditions as in Example 1, to measure the powder X ray diffraction pattern.

The results of this measurement are shown in Fig. 5 and Table 6.

**Table 6**

| Comparison | Diffraction Angle 2θ (°) | Relative intensity |
|---|---|---|
| ○ | 11.40 | 100 |
| ○ | 16.10 | 37 |
| | 18.38 | 27 |
| | 19.78 | 51 |
| | 21.89 | 23 |
| | 22.17 | 14 |
| | 23.48 | 14 |
| | 24.67 | 10 |
| | 27.86 | 16 |
| | 31.59 | 71 |

In Table 6, a peak appended with "○ mark" in the column of comparison denotes a peak corresponding to a peculiar peak of a B type crystal of a compound (I), however, no peculiar peak of a B type crystal was observed at other diffraction angles, and it is difficult to judge whether the crystal of a compound (I) prepared under this condition is a B type crystal or not.

Further, a chart of an infrared absorption spectrum of the above-mentioned novel crystal obtained here is shown in Fig. 6.
IR (Nujol): 1770, 1665, 1460 cm⁻¹

### INDUSTRIAL APPLICABILITY

An anhydrous form or mono-hydrate of a B type crystal of the compound (I) of the present invention is not bulky, has very high crystal purity, extremely stable against heat, light and the like, and simultaneously, shows excellent solubility in water, therefore, formulation thereof is easy, and use as a medical preparation is suitable, and handling in preparation and preservation is also easy.

Further, the anhydrous form or mono-hydrate of a B type crystal of a compound (I) has sufficiently large filtration speed, also has a feature that working on preparation can be conducted very efficiently, and is useful for a medical preparation.

## Claims

1. A novel crystal of 7-[2-(2-aminothiazol-4-yl)-2-hydroxyiminoacetamide]-3-vinyl-3-cephem-4-carboxylic acid (syn isomer), **characterized in that** it exhibits peaks at diffraction angles shown in the following Table 1, in its powder X ray diffraction pattern:

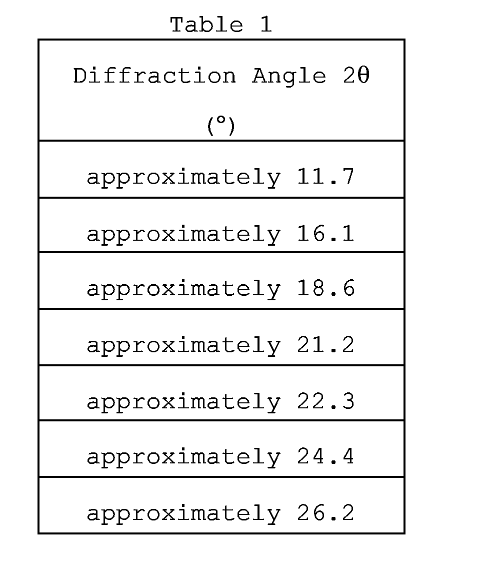

2. The novel crystal of 7-[2-(2-aminothiazol-4-yl)-2-hydroxyiminoacetamide]-3-vinyl-3-cephem-4-carboxylic acid (syn isomer) according to Claim 1, obtained by crystallization in an acidic state of a solution containing 7-[2-(2-aminothiazol-4-yl)-2-hydroxyiminoacetamide]-3-vinyl-3-cephem-4-carboxylic acid (syn isomer) in a temperature range of -5°C to 5°C.

3. The novel crystal of 7-[2-(2-aminothiazol-4-yl)-2-hydroxyiminoacetamide]-3-vinyl-3-cephem-4-carboxylic acid (syn isomer) according to Claim 1 or 2, wherein the solution containing 7-[2-(2-aminothiazol-4-yl)-2-hydroxyiminoacetamide]-3-vinyl-3-cephem-4-carboxylic acid (syn isomer) is an aqueous solution of an alkali metal salt of the compound.

4. The novel crystal of 7-[2-(2-aminothiazol-4-yl)-2-hydroxyiminoacetamide]-3-vinyl-3-cephem-4-carboxylic acid (syn isomer) according to any of Claims 1 to 3, obtained by controlling pH of an aqueous sodium hydrogen carbonate solution of 7-[2-(2-aminothiazol-4-yl)-2-hydroxyiminoacetamide]-3-vinyl-3-cephem-4-carboxylic acid (syn isomer) at from 1 to 3 while cooling the solution in a temperature range from -5°C to 5°C.

5. A method for preparing a novel crystal of 7-[2-(2-aminothiazol-4-yl)-2-hydroxyiminoacetamide]-3-vinyl-3-cephem-4-carboxylic acid (syn isomer), comprising acidifying a solution containing 7-[2-(2-aminothiazol-4-yl)-2-hydroxyiminoacetamide]-3-vinyl-3-cephem-4-carboxylic acid (syn isomer) in a temperature range from -5°C to 5°C to cause formation of a crystal.

6. The method for preparing a novel crystal of 7-[2-(2-aminothiazol-4-yl)-2-hydroxyiminoacetamide]-3-vinyl-3-cephem-4-carboxylic acid (syn isomer) according to Claim 5, wherein the acidic state of the solution includes pH values of 1 to 3.

7. The method for preparing a novel crystal of 7-[2-(2-aminothiazol-4-yl)-2-hydroxyiminoacetamide]-3-vinyl-3-cephem-4-carboxylic acid (syn isomer) according to Claim 5 or 6, wherein the solution containing 7-[2-(2-aminothiazol-4-yl)-2-hydroxyiminoacetamide]-3-vinyl-3-cephem-4-carboxylic acid (syn isomer) is an aqueous solution of an alkali metal salt of the compound.

8. The method for preparing a novel crystal of 7-[2-(2-aminothiazol-4-yl)-2-hydroxyiminoacetamide]-3-vinyl-3-cephem-4-carboxylic acid (syn isomer) according to any of Claims 5 to 7, wherein the temperature of the solution under an acidic state is 0°C to 2°C.

9. A method for preparing an anhydrous form of a novel crystal of 7-[2-(2-aminothiazol-4-yl)-2-hydroxyiminoacetamide]-3-vinyl-3-cephem-4-carboxylic acid (syn isomer), wherein a novel crystal obtained by the method according to any of Claims 5 to 8 is further frozen at temperatures from -5°C to -80°C, and then subjected to vacuum drying.

10. The method for preparing an anhydrous form of a novel crystal of 7-[2-(2-aminothiazol-4-yl)-2-hydroxyiminoacetamide]-3-vinyl-3-cephem-4-carboxylic acid (syn isomer) according to Claim 9, wherein the conditions for vacuum drying include a degree of vacuum of 0.1 to 0.001 mmHg and a temperature of -20 to 35°C.
